# EUROPEAN PATENT APPLICATION

(11) **EP 4 803 185 A1**
(43) Date of publication of application: **09.09.2026**
(21) Application number: 26158775.2
(22) Date of filing: 16.02.2026
(51) Int. Cl.: B01J 4/00, B01J 19/18, B01J 3/04, B01J 12/00, C07C 1/04, C07C 1/12, C10L 3/08

(54) **CONDUCTING AN EXOTHERMIC METHANATION REACTION OF CARBON OXIDES**

(30) Priority: 14.02.2025 PL 45121325
(71) Applicant: Centrum Badan i Rozwoju Technologii dla Przemyslu S.A., 02-679 Warszawa (PL)
(72) Inventor: Putynkowski, Grzegorz, Audley, ST78EA (GB); Socha, Robert, 32-091 Zerwana (PL); Wojnicki, Marek, 32-085 Modlnica (PL)
(74) Representative: Kancelaria Eupatent.pl Sp. z o.o.

(57) **Abstract**

A reactor for conducting an exothermic methanation reaction of carbon oxides, comprising: a cylinder (14, 24) defining a reaction chamber of variable volume and having at least one inlet channel (161, 171, 172, 261, 262) for introducing into the cylinder (14, 24) gaseous reactants of the methanation reaction, namely hydrogen and carbon monoxide and/or carbon dioxide, and at least one outlet channel (18, 281, 282) for removing reaction products from the cylinder (14, 24), a catalyst arranged to catalyse the exothermic methanation reaction of said reactants in the cylinder (14, 24), at least one piston (11, 211, 212) having a piston crown (111, 213, 214) and being arranged for reciprocating movement in the cylinder (14, 24) to vary the volume of the reaction chamber, and at least one crankshaft (13, 231, 232) mechanically coupled to the piston (11, 211, 212), wherein the mechanical coupling is configured such that, in use, rotation of the crankshaft (13, 231, 232) drives reciprocating movement of the piston (11, 211, 212) in a direction decreasing the volume of the reaction chamber to compress the reactants, and such that exothermic methanation in the cylinder (14, 24) causes an increase in pressure and expansion of gases in the reaction chamber which drives the piston (11, 211, 212) in a direction increasing the volume of the reaction chamber and thereby drives rotation of the crankshaft (13, 231, 232) so as to convert heat released by the methanation reaction into mechanical energy.

## Description

### TECHNICAL FIELD

The subject of the invention is a reciprocating piston-cylinder reactor for conducting an exothermic methanation reaction of carbon oxides and a method for conducting an exothermic methanation reaction using this reactor to ensure recovery of the energy released in the reaction.

### BACKGROUND

Methanation is a catalytic reaction in which carbon oxides, in particular carbon monoxide (CO) and/or carbon dioxide (CO₂), react with hydrogen to form methane and water. Methanation is employed, for example, for producing synthetic methane, for removing residual carbon oxides from process gas streams, and in "Power-to-Gas" concepts in which hydrogen produced by electrolysis is converted to methane for storage and transport in existing gas infrastructure.

A variety of catalysts have been proposed and used for methanation, including supported metal catalysts based on nickel, ruthenium and palladium. Nickel-based catalysts are widely applied due to their activity and selectivity toward methane, but may be susceptible to deactivation mechanisms such as carbon deposition depending on operating conditions and feed composition. Noble-metal catalysts, including ruthenium and palladium, are also known and may exhibit different activity, selectivity and stability characteristics depending on the support material and the process regime.

The methanation of CO and CO₂ is strongly exothermic. As a consequence, temperature management is an important consideration in reactor and process design, particularly to limit local temperature excursions, to maintain catalyst performance over time, and to operate within desired conversion and selectivity windows. Conventional methanation processes typically employ continuous reactors, for example fixed-bed arrangements, optionally in multiple stages with intermediate cooling, or reactor configurations incorporating internal or external heat exchange surfaces. Heat released by the reaction may be removed by cooling media and can be recovered to some extent for use elsewhere in a plant, for example for preheating reactants or producing steam, depending on integration constraints and the available temperature levels.

The patent literature also describes systems in which the energy released by methanation is coupled to auxiliary equipment. For example, WO2022078747 describes a system in which products from a methanation reactor are supplied to a turbocharger via a gas line such that the energy associated with the reaction products is used to drive the turbocharger.

Despite these developments, there remains a need for reactor systems and methanation process concepts that provide effective temperature control while enabling improved recovery and utilisation of the heat generated by the exothermic reaction, in order to increase overall process efficiency and reduce energy losses in practical implementations.

### SUMMARY

It would therefore be desirable to provide a new reactor design for the catalytic conversion of carbon oxides (CO and CO₂), hereinafter "COx", to hydrocarbons, such as methane, that enables more efficient utilization of the heat released in the methanation reaction.

This objective is achieved by the new design of a reciprocating piston-cylinder reactor and by a method for conducting the methanation reaction in said reactor, whereby the heat released by the exothermic reaction is used directly to generate mechanical energy. In particular, the reactor design enables efficient conversion of chemical energy into mechanical energy, and can reduce or eliminate the need for dedicated cooling systems that are typically required in conventional flow reactors.

The new reactor design enables efficient use of the reaction heat for generating mechanical energy. In particular, by an appropriate combination of mechanical and chemical means, a dynamic change in the volume of the reaction chamber is provided, thereby allowing the thermal energy produced as a by-product of the methanation reaction to be harnessed. The reactor enables the methanation reaction to proceed under advantageous pressure conditions, which can improve conversion of reactants. Compression and expansion of the reactants also support control of the reaction temperature, reducing heat losses and increasing overall efficiency. At the same time, the heat generated by the reaction is converted into mechanical energy in the reactor.

The reactor can be implemented as a compact unit, facilitating integration with other systems, for example fuel cells, energy generators, or gas storage systems. Owing to its modular design, the reactor can be readily scaled to suit different needs, ranging from small mobile units to large industrial systems.

The reactor design presented herein makes it possible to efficiently convert CO₂ into hydrocarbons, in particular methane, which can be used as a fuel. The method for converting COx to hydrocarbons presented herein can be implemented in greenhouse gas emission reduction processes and in technologies using waste sources of carbon dioxide, for example from the power or chemical industry.

The reactor comprises working elements that together enable both the chemical process and conversion of chemical energy into mechanical energy. Each of these elements performs a specific function and has design features adapted to operation under elevated temperature and pressure conditions.

The cylinder has inlet channels and outlet channels that allow introduction of reactants into the reaction space of the cylinder and removal of reaction products. This enables regulation of the supply of gaseous reactants, supports maintenance of an appropriate pressure in the system, and ensures distribution of the gas mixture in the cylinder. With a suitable arrangement of the inlet channels, advantageously axially and/or radially, efficient mixing of the reactants can be achieved. To increase flow precision, the inlet and outlet channels may be equipped with control valves.

Optionally, the cylinder may be provided with at least one channel for supplying an auxiliary energy input, allowing energy to be introduced into the cylinder in the form of electromagnetic irradiation or heat. This can support the methanation reaction and enable more precise control of process conditions. The channel may be made of high-temperature metal alloys or insulating ceramics, and an alternative design may include a waveguide for delivering microwave radiation into the reactor.

The piston, performing a reciprocating motion, provides regulation of the cylinder volume, enabling compression and expansion of the reactants, which directly affects the reaction conditions. The piston may have its piston crown coated with a catalyst to increase process efficiency.

The crankshaft is driven by the reciprocating motion of the piston and enables conversion of thermal energy, generated as a by-product of the methanation reaction, into mechanical energy. This makes it possible to store or directly utilize the energy generated by the reaction. A crankshaft design with variable geometry can use rotational inertia to regulate the dynamics of piston movement.

The connecting rod movably connects the piston to the crankshaft, ensuring transfer of force between the piston and the crankshaft and conversion of linear motion into rotary motion. Optionally, the connecting rod may have an adjustable length, allowing the compression ratio of the reactor to be changed, advantageously during operation, for example in response to changes in the concentration of reactants supplied to the reactor cylinder.

The crankshaft chamber constitutes the space for rotary movement of the crankshaft. Optionally, the crankshaft chamber may be equipped with a cooling system, for example oil cooling, to increase durability of the mechanism.

The catalyst may be deposited on the walls of the cylinder and/or on the piston crown of the piston(s), providing the desired selectivity of the reaction. As catalysts for the methanation reaction of COx, nickel, ruthenium and/or palladium, as well as alloys of these metals, may be used.

### BRIEF DESCRIPTION OF DRAWINGS

The invention is illustrated by way of example embodiments in the accompanying drawings, in which:
Fig. 1A is a front view, in longitudinal section, of a reactor for conversion of carbon oxides according to a first example embodiment.
Fig. 1B is an axonometric view, in longitudinal section, of the reactor of Fig. 1A.
Fig. 2A is a front view, in longitudinal section, of a reactor for conversion of carbon oxides according to a second example embodiment.
Fig. 2B is an axonometric view, in longitudinal section, of the reactor of Fig. 2A.
Fig. 2C is a cross-sectional view of the reactor of Fig. 2A-2B.
Fig. 3A shows thermodynamic parameters of the methanation reaction of carbon monoxide in relation to temperature.
Fig. 3B shows thermodynamic parameters of the methanation reaction of carbon dioxide in relation to temperature.
Fig. 4 is a block diagram illustrating phases of operation of the process for conversion of carbon oxides to methane using the reactor.

### DETAILED DESCRIPTION

The reactor operates according to a Carnot cycle. However, instead of combusting fuel with oxygen, the reactor according to the invention is configured to carry out an exothermic methanation reaction, and the reactor design enables conversion of the thermal energy released by the reaction into mechanical energy.

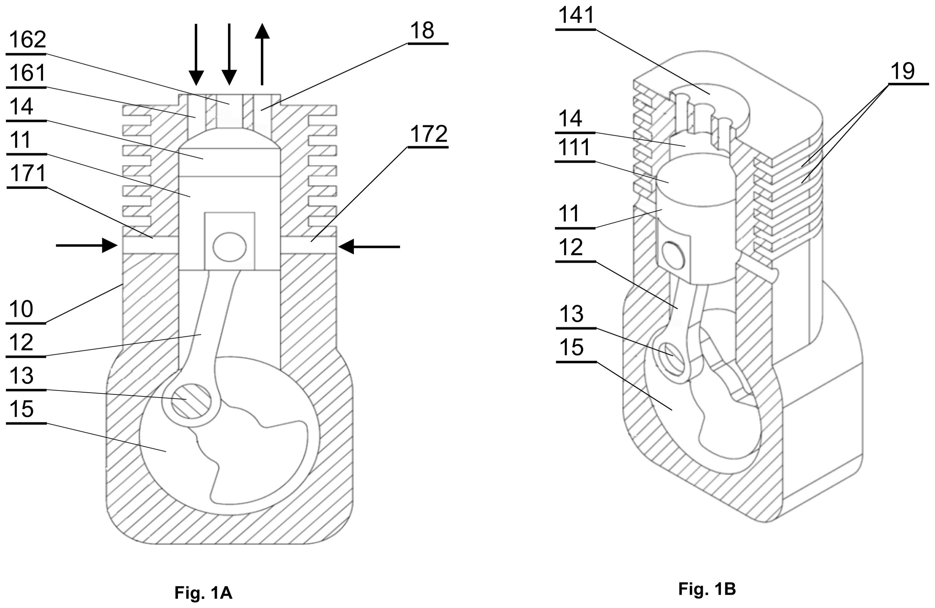

### First example embodiment (Fig. 1A, 1B)

In Fig. 1A and Fig. 1B, a reactor 10 according to the invention is shown in a first example embodiment, in longitudinal section with the structure visible, in front view (Fig. 1A) and in axonometric view (Fig. 1B).

The reactor 10 comprises a piston 11 connected via a connecting rod 12 to a crankshaft 13, and a cylinder 14 terminated by a crankshaft chamber 15. The piston 11 is associated with the cylinder 14 such that, during operation of the reactor 10, the piston 11 performs a reciprocating motion within the cylinder 14, thereby producing rotary motion of the crankshaft 13 in the crankshaft chamber 15.

The cylinder 14 constitutes a reaction chamber, and the crankshaft chamber 15 constitutes a space for rotation of the crankshaft 13. The walls of the cylinder 14 and/or the front surface (piston crown) 111 of the piston 11 are coated with a catalytically active layer (washcoat) comprising a layer of catalyst, advantageously a nickel (Ni), ruthenium (Ru), or palladium (Pd) catalyst, or an alloy of these metals, advantageously on a refractory oxide such as alumina (Al₂O₃), silicon oxide (SiO₂), and/or aluminosilicate (SiO₂-Al₂O₃), in order to increase catalyst surface area and ensure the proper course of the reaction with catalyst participation. In addition, the front surface (piston crown) 111 of the piston 11 may be further coated with a susceptor layer for absorbing an auxiliary energy input, such as, for example, tungsten carbide (WC), silicon carbide (SiC), or germanium nitride (Ge₃N₄).

The reactor 10 has an inlet channel 161 in a head 141 of the cylinder 14 and inlet channels 171, 172 arranged circumferentially in a wall of the cylinder 14, for introducing reactants into the interior of the cylinder 14. The reactor 10 further has an outlet channel 18 in the head 141 of the cylinder 14 for removing reaction products from the cylinder 14. Optionally, the cylinder 14 may further comprise an additional inlet channel 162 in the head 141 for introducing an auxiliary energy input into the cylinder 14 in order to increase the reaction rate.

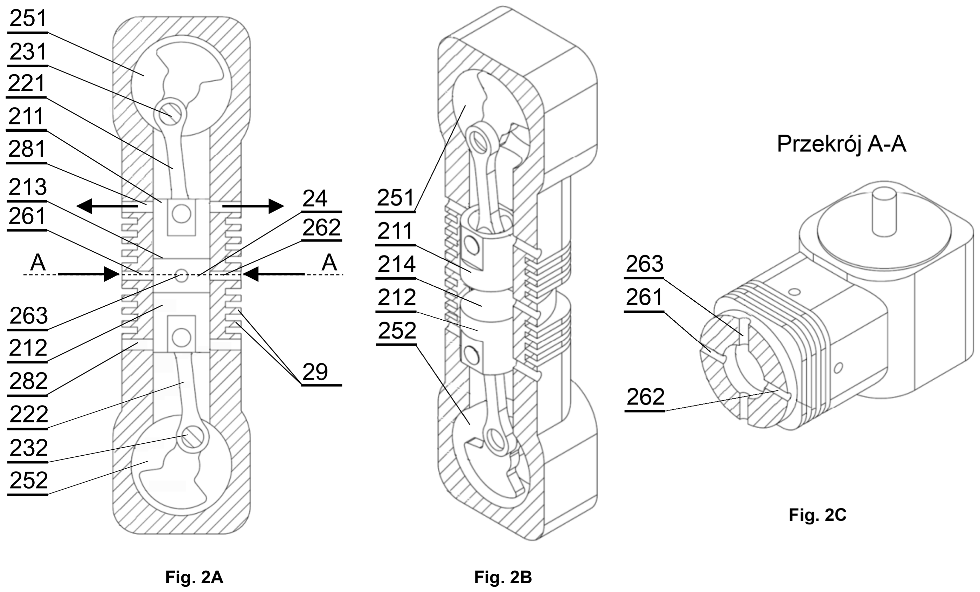

### Second example embodiment (Fig. 2A-2C)

In Fig. 2A and Fig. 2B, a reactor 20 according to the invention is shown in a second example embodiment, in longitudinal section with the structure visible, in front view (Fig. 2A) and in axonometric view (Fig. 2B). In Fig. 2C, the reactor 20 is shown in cross-section.

The reactor 20 comprises two pistons 211, 212, each connected via a respective connecting rod 221, 222 to a respective crankshaft 231, 232, and a cylinder 24 terminated on opposite sides by crankshaft chambers 251, 252. Each piston 211, 212 is associated with the cylinder 24 such that, during operation of the reactor 20, each piston 211, 212 performs a reciprocating motion in the cylinder 24, thereby producing rotary motion of the associated crankshaft 231, 232 in the respective crankshaft chamber 251, 252. The pistons 211, 212 operate in a coordinated manner, either in an opposed-piston arrangement or in an opposed-piston arrangement with a phase offset (described in more detail below), resulting in a more uniform load distribution in the structure.

The cylinder 24 constitutes a reaction chamber, and the crankshaft chambers 251, 252 constitute spaces for rotation of the crankshafts 231, 232. The walls of the cylinder 24 and/or the front surfaces (piston crowns) 213, 214 of the pistons 211, 212 are coated with a catalytically active layer (washcoat) comprising a layer of catalyst, advantageously Ni, Ru, Pd, or an alloy of these metals, advantageously on a refractory oxide such as alumina (Al₂O₃), silicon oxide (SiO₂), and/or aluminosilicate (SiO₂-Al₂O₃), in order to increase catalyst surface area and ensure the proper course of the reaction with catalyst participation. In addition, the front surfaces (piston crowns) 213, 214 may be further coated with a susceptor layer for absorbing an auxiliary energy input, such as, for example, tungsten carbide (WC), silicon carbide (SiC), or germanium nitride (Ge₃N₄).

The reactor 20 has radial inlet channels 261 and 262 for supplying reactants to the interior of the cylinder 24, arranged circumferentially in one cross-section of the cylinder 24, advantageously in a central cross-section between the first piston 211 and the second piston 212. The reactor 20 further has radial outlet channels 281 and 282 for removing products from the cylinder 24, advantageously arranged circumferentially in two cross-sections of the cylinder 24. Optionally, the cylinder 24 may further comprise an additional radial inlet channel 263 for introducing an auxiliary energy input into the cylinder 24 to increase the reaction rate, advantageously located circumferentially together with the inlet channels 261, 262 for the reactants.

### Optional cooling and ventilation features

Furthermore, the crankshaft chamber 15, 251, 252 of the reactor 10, 20 may be ventilated to prevent overheating of the mechanical system. Optionally, the reactor 10, 20 may comprise cooling fins 19, 29 on the outer side walls along the cylinder 14, 24, increasing the heat exchange surface between the cylinder interior and the environment and providing additional cooling.

Advantageously, the reactor 10, 20 is capable of compressing gaseous reactants in the range of 3-18 bar, and more advantageously in the range of 5-154 bar, with a compression ratio in the range of 6:1 to 20:1, and more advantageously with a compression ratio in the range of 11:1 to 15:1.

### Principle of operation and reaction products

The operating principle of the reactor 10, 20 is as follows. During the motion of the piston 11, or the pistons 211, 212, a mixture of gaseous reactants in the cylinder 14, 24 is compressed, which initiates an exothermic methanation reaction and converts released energy into mechanical energy. This conversion results from a rapid increase in the volume of the product gas mixture and its expansion, which drives the piston(s) 11, 211, 212 in the opposite direction. The reaction involves conversion of carbon oxides, namely carbon monoxide (CO) and/or carbon dioxide (CO₂), into gaseous hydrocarbons, advantageously methane (CH₄), and formation of water vapour (H₂O). Under appropriate catalysts and reaction conditions, minor amounts of higher hydrocarbons may also be formed.

The composition of the product gas mixture depends on the catalyst type and the reaction conditions. For example, methane (CH₄) is obtained in the presence of Ni or Ru catalysts. Minor amounts of C₂ hydrocarbons such as ethane (C₂H₆) and/or ethene (C₂H₄) may occur as byproducts under appropriate conditions. Minor amounts of C₃ and C₃+ hydrocarbons such as propanes and butanes (C₃H₈, C₄H₁₀) may be formed under appropriate conditions. In particular, nickel (Ni) shows high selectivity to methane. Ruthenium (Ru), like Ni, shows high selectivity to methane, while also being able to promote formation of higher hydrocarbons under suitable temperature and pressure conditions. Palladium (Pd) shows lower selectivity than Ni and Ru and can promote formation of short alkanes and alkenes (C₂-C₄) and/or trace oxygenates (e.g., methanol).

In the reactor 10, 20, the process occurs on the surface of a catalyst, advantageously Ni, Ru or Pd, or an alloy of two or three of Ni, Ru and Pd, which covers the walls of the cylinder 14, 24 and/or the front surfaces (piston crowns) 111, 213, 214. The reaction is strongly exothermic, and the heat generated is used directly to drive the piston(s) 11, 211, 212 and thus the crankshaft(s) 13, 231, 232.

The methanation reactions occurring in the cylinder 14, 24, namely methanation of carbon monoxide (CO) and carbon dioxide (CO₂), are as follows:

Reaction Scheme 1: CO + 3H₂ → CH₄ + H₂O

The standard enthalpy of methanation of carbon monoxide according to Reaction Scheme 1 is -206 kJ/mol.

Reaction Scheme 2: CO₂ + 4H₂ → CH₄ + 2H₂O

The standard enthalpy of methanation of carbon dioxide according to Reaction Scheme 2 is -165 kJ/mol.

In the reactor design 10, 20, a gaseous mixture of reactants (H₂ and CO and/or CO₂) is supplied to the cylinder 14, 24 through the inlet channels 161, 171, 172, 261, 262. The gases in the cylinder 14, 24 are then compressed by the piston 11, or pistons 211, 212, which increases temperature and improves contact with the catalyst. On the catalyst surface, adsorption of the reactants occurs (H₂ and CO and/or CO₂). Hydrogen H₂ is activated, forming reactive atoms which then combine with carbon oxides to form methane (CH₄) and/or higher hydrocarbons, and water vapour (H₂O). The heat generated causes a rapid pressure increase in the cylinder 14, 24, leading to expansion of the gases and displacement of the piston 11, or pistons 211, 212, toward the respective crankshaft chambers 15, 251, 252. After completion of the cycle, gaseous products (CH₄ and/or higher hydrocarbons and H₂O) are removed via the outlet channels 18, 281, 282.

### Process conditions and auxiliary energy input

According to the method presented herein, the methanation reactions in the reactor 10, 20 according to Reaction Scheme 1 and/or Reaction Scheme 2 are carried out while ensuring a pressure in the cylinder 14, 24 in the range from 1 to 30 bar, and more advantageously 5-30 bar. When using a nickel catalyst, the process is advantageously carried out at a pressure in the range of 10-30 bar, and when using a ruthenium catalyst, the process is advantageously carried out at a pressure in the range of 5-20 bar. Ensuring sufficiently high pressures in the cylinder 14, 24, advantageously in the range of 20-30 bar, shifts the reaction equilibrium to the right (toward the products), which further increases process efficiency.

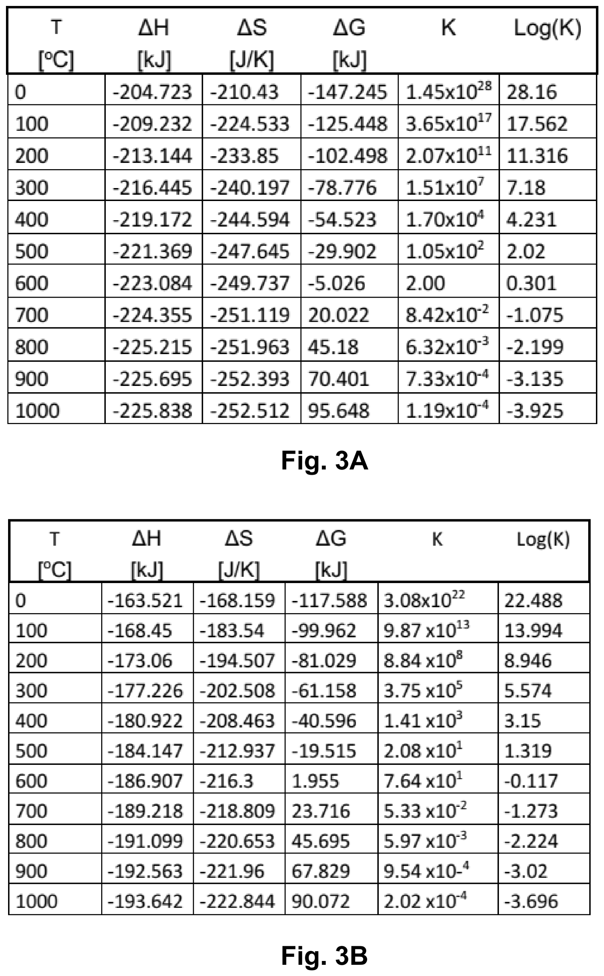

The process is carried out by maintaining the temperature in the cylinder in the range from 0 to 2000°C, and more advantageously in the range 200-600°C, wherein the pressure of the supplied reactants may be from 1 to 3000 bar. The temperature in the cylinder 14, 24 may be increased (within a range not exceeding 2000°C) by an auxiliary energy input supplied through the inlet channel 162 or 263 of the reactor 10, 20. In Fig. 3A, a table lists values of the change in enthalpy ΔH, entropy ΔS, Gibbs free energy ΔG, and equilibrium constant K and log(K) for the reaction involving carbon monoxide (Reaction Scheme 1) at given temperatures. In Fig. 3B, a table lists the corresponding values for the reaction involving carbon dioxide (Reaction Scheme 2).

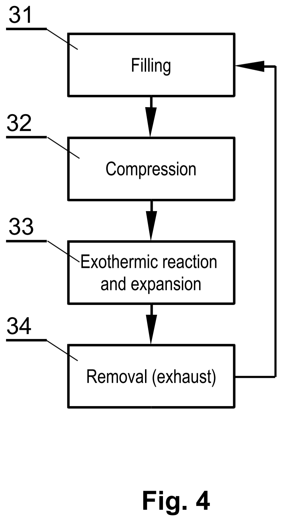

### Operating cycle (Fig. 4)

In Fig. 4, the phases of operation of the reactor 10, 20 are shown as a block diagram. The reactor 10, 20 operates on the principle of reciprocating motion, in which each piston 11, 211, 212 reciprocates in the cylinder 14, 24. The piston motion is transmitted via the connecting rod 12, 221, 222 to the crankshaft 13, 231, 232, which rotates in the crankshaft chamber 15, 251, 252.

The operating cycle of the reactor 10, 20 comprises: a filling phase (stage 31), in which reactants are supplied to the cylinder 14, 24; a compression phase (stage 32); an exothermic reaction and expansion phase (stage 33); and a removal (exhaust) phase of reaction products (stage 34).

In stage 31, the piston 11, 211, 212 is in an upper position (top dead centre, TDC), which in the single-piston reactor 10 corresponds to the piston 11 being close to the head 141 of the cylinder 14, and in the two-piston reactor 20 corresponds to an analogous position of the pistons 211, 212, namely the extreme upper position of at least one of the two pistons. In this stage, the volume of the cylinder 14, 24 on the side of the front surface (piston crown) 111, 213, 214 is smallest. Alternatively, in stage 31 the piston(s) 11, 211, 212 may be in an intermediate position between TDC and the extreme lower position (bottom dead centre, BDC).

In the two-piston reactor 20, the pistons 211, 212 may operate in an opposed-piston arrangement (mirror movements) or in an opposed-piston arrangement with a phase offset, in which the pistons 211, 212 do not reach their extreme positions at exactly the same moment but with a slight delay, thereby achieving a smoother change in the cylinder volume.

In stage 31, each crankshaft 13, 231, 232 rotates in the crankshaft chamber 15, 251, 252 and moves the piston 11, 211, 212 toward the respective crankshaft chamber 15, 251, 252, thereby increasing cylinder volume and creating reduced pressure inside the cylinder 14, 24 which constitutes the reaction chamber. As a result of this piston movement, the relevant inlet channels 161, 171, 172, 261, 262 are uncovered, through which a reactant mixture of carbon monoxide and/or carbon dioxide and hydrogen is supplied to the cylinder 14, 24. Optionally, at this stage an auxiliary energy input, e.g., electromagnetic irradiation such as microwaves, laser light or LED light, and/or heat such as infrared radiation, may be introduced into the cylinder 14, 24 through the additional axial inlet channel 162 or radial inlet channel 263, to activate the catalyst and/or increase reaction efficiency.

Next, in stage 32, the direction of movement of each piston 11, 211, 212 changes. The piston 11 moves toward the head 141 of the cylinder 14, or the pistons 211, 212 approach each other. The connecting rod 12, 221, 222 transmits force to the crankshaft 13, 231, 232, which continues rotating in the crankshaft chamber 15, 251, 252. As the crankshaft rotation drives the piston(s), the reactants in the cylinder 14, 24 are compressed due to a decrease in the cylinder volume, resulting in an increase in pressure and temperature. In the presence of the catalyst, this initiates the methanation reaction.

Next, in stage 33, the piston 11 moving toward the head 141 of the cylinder 14, or the pistons 211, 212 approaching each other, reaches/reach the maximum upper position(s) (TDC). In the cylinder 14, 24, carbon monoxide (CO) and/or carbon dioxide (CO₂) reacts with hydrogen, producing methane and possibly higher hydrocarbons (as described above), and water vapour. The reaction is strongly exothermic and causes a rapid increase in pressure and temperature in the cylinder 14, 24. The resulting expansion of gases rapidly increases cylinder volume and displaces the piston(s) 11, 211, 212 away from the direction of the respective crankshaft chamber 15, 251, 252. The connecting rod 12, 221, 222 transmits the resulting force to the crankshaft 13, 231, 232, driving further rotary motion. The mechanical work thus generated can be used, for example, to generate electricity as described below.

In stage 34, each piston 11, 211, 212 reaches its maximum lower position (BDC) at the crankshaft chamber 15, 251, 252 and begins to move in the opposite direction: the piston 11 toward the head 141 of the cylinder 14, and the pistons 211, 212 toward each other. At this stage, the relevant outlet channel 18, 281, 282 opens, through which the reaction products are removed from the cylinder 14, 24. At the same time, each piston 11, 211, 212 continues moving and the crankshaft 13, 231, 232 rotates, thereby preparing the system for the next operating cycle.

After the products are removed from the cylinder 14, 24, stage 31 begins again, initiating the next intake of reactants through the inlet channels 161, 171, 172, 261, 262.

### Utilisation of the generated mechanical energy

The crankshaft 13, 231, 232 of the reactor 10, 20, driven by the exothermic reaction converting carbon oxides to hydrocarbons, in particular methane, in the cylinder 14, 24, may be associated with various devices for utilising the energy released by the reaction, which is converted in the reactor 10, 20 into mechanical energy. For example, the crankshaft 13, 231, 232 may be associated with a generator for converting mechanical energy into electrical energy, in which crankshaft rotation is transmitted to a rotor shaft of the generator to produce electric current. Alternatively, the crankshaft may be associated with a compressor (e.g., a piston, screw, or vane compressor) for compressing air or process gases such as methane CH₄ or hydrogen H₂.

The crankshaft 13, 231, 232 may also be associated with a hydraulic pump (e.g., a piston, gear, or vane pump) for generating high hydraulic-fluid pressures, or with a mechanical turbine, or with a flywheel for accumulating kinetic energy. Further, the crankshaft may be used for driving vehicles or transport devices, for example industrial or agricultural machines, in which the crankshaft 13, 231, 232 is connected to a mechanical transmission and differential system that transmits torque to vehicle wheels or drives a conveyor belt.

### Detailed embodiment

In one example embodiment, a gaseous mixture comprising 0.005 mole of CO₂ and 0.01 mole of H₂ was introduced through an inlet channel into the cylinder of a single-piston reactor of the type shown in Fig. 1. The reactor had a piston diameter of 76 mm, a maximum cylinder volume of 0.3 L, and a piston stroke of 82 mm. The geometric compression ratio of the reactor was 6.5, with a pressure ratio of 9.

At the inlet to the cylinder, the pressure of the reactants was maintained at 2 bar, as a result of the reciprocating movement of the piston increasing the cylinder volume due to rotary motion of the crankshaft. The reactants were subsequently compressed in the cylinder by the reciprocating movement of the piston decreasing the cylinder volume, likewise driven by rotation of the crankshaft. During compression, microwave irradiation was applied (source frequency 2.4 GHz and power in the range of 100-900 W), thereby causing an exothermic reaction and expansion of the reactants in the cylinder. The resulting expansion displaced the piston in the direction increasing the cylinder volume and forced continued rotary motion of the crankshaft.

A Pd catalyst on an Al₂O₃ support was used, with the catalyst located on the active surface of the piston. During the process, the temperature of the reactants in the cylinder ranged from 30°C (at the inlet to the cylinder) to 200°C (immediately after the exothermic reaction). The pressure in the cylinder varied from 2 bar during intake of the reactants to a maximum value of 18 bar during the compression phase.

As a result of a single operating cycle of the reactor, a product gas mixture was obtained having the following composition: 0.2 moles of CH₄, 2 moles of methanol, and 6.5 moles of H₂O.

In this manner, the obtained reactor power at a rotational speed of 1000 rpm was 1.6 kW.

## Claims

1. A reactor for conducting an exothermic methanation reaction of carbon oxides, comprising:
- a cylinder (14, 24) defining a reaction chamber of variable volume and having at least one inlet channel (161, 171, 172, 261, 262) for introducing into the cylinder (14, 24) gaseous reactants of the methanation reaction, namely hydrogen and carbon monoxide and/or carbon dioxide, and at least one outlet channel (18, 281, 282) for removing reaction products from the cylinder (14, 24),
- a catalyst arranged to catalyse the exothermic methanation reaction of said reactants in the cylinder (14, 24),
- at least one piston (11, 211, 212) having a piston crown (111, 213, 214) and being arranged for reciprocating movement in the cylinder (14, 24) to vary the volume of the reaction chamber, and
- at least one crankshaft (13, 231, 232) mechanically coupled to the piston (11, 211, 212),
- wherein the mechanical coupling is configured such that, in use, rotation of the crankshaft (13, 231, 232) drives reciprocating movement of the piston (11, 211, 212) in a direction decreasing the volume of the reaction chamber to compress the reactants, and such that exothermic methanation in the cylinder (14, 24) causes an increase in pressure and expansion of gases in the reaction chamber which drives the piston (11, 211, 212) in a direction increasing the volume of the reaction chamber and thereby drives rotation of the crankshaft (13, 231, 232) so as to convert heat released by the methanation reaction into mechanical energy.

2. The reactor according to claim 1, wherein the catalyst is deposited on an inner wall of the cylinder (14, 24) and/or on the piston crown (111, 213, 214) of the piston (11, 211, 212).

3. The reactor according to claim 1 or 2, wherein the crankshaft (13, 231, 232) is connected to the piston (11, 211, 212) by a connecting rod (12, 221, 222), and wherein the cylinder (14, 24) is terminated by at least one crankshaft chamber (15, 251, 252) accommodating the crankshaft (13, 231, 232).

4. The reactor according to any of the preceding claims, further comprising at least one energy-input channel (162, 263) for introducing an external energy input into the cylinder (14, 24).

5. The reactor according to any of claims 1 to 4, comprising a single piston (11), wherein the cylinder (14) has a head (141) comprising an inlet channel (161) and an outlet channel (18), and wherein the cylinder (14) further comprises inlet channels (171, 172) arranged circumferentially in a wall of the cylinder (14).

6. The reactor according to any of claims 1 to 4, comprising two pistons (211, 212), each piston (211, 212) being connected to a respective crankshaft (231, 232), wherein the cylinder (24) is terminated on opposite sides by crankshaft chambers (251, 252).

7. The reactor according to claim 6, wherein the cylinder (24) comprises inlet channels (261, 262) and outlet channels (281, 282) arranged circumferentially in a wall of the cylinder (24).

8. The reactor according to claims 4 and 5, wherein the energy-input channel (162) is provided in the head (141) of the cylinder (14).

9. The reactor according to claims 4 and 6, wherein the energy-input channel (263) is provided in the wall of the cylinder (24).

10. A method for conducting an exothermic methanation reaction of carbon oxides, in which carbon monoxide and/or carbon dioxide is reacted with hydrogen in the presence of a catalyst to obtain methane and water vapour, the method being carried out in a reactor according to any of claims 1 to 9 and comprising, in an operating cycle:
- introducing reactants into the cylinder (14, 24) through said at least one inlet channel (161, 171, 172, 261, 262) during an intake phase in which the piston (11, 211, 212) increases the volume of the reaction chamber,
- compressing the reactants in the cylinder (14, 24) during a compression phase in which the piston (11, 211, 212) decreases the volume of the reaction chamber, driven by rotation of the crankshaft (13, 231, 232),
- carrying out exothermic methanation in the cylinder (14, 24) so that the associated pressure increase and gas expansion drive the piston (11, 211, 212) to increase the volume of the reaction chamber and thereby drive rotation of the crankshaft (13, 231, 232), and
- removing reaction products from the cylinder (14, 24) through said at least one outlet channel (18, 281, 282) during an exhaust phase.

11. The method according to claim 10, wherein the catalyst comprises nickel (Ni), ruthenium (Ru) and/or palladium (Pd) and/or an alloy of two or three metals selected from the group consisting of Ni, Ru and Pd.

12. The method according to claim 10 or 11, wherein the pressure of the reactants at an inlet to the cylinder (14, 24) is maintained in the range from 1 to 3000 bar, and wherein the temperature in the cylinder (14, 24) is maintained in the range from 0 to 2000°C.

13. The method according to any one of claims 10 to 12, wherein an external energy input is introduced into the cylinder (14, 24) via the energy-input channel (162, 263), the external energy input comprising electromagnetic radiation having a wavelength in the range from 1.5 × 10⁻⁷ m to 100 m, covering the ultraviolet (UV), visible (VIS), near infrared (NIR), mid-infrared (MIR), far infrared (FIR) and microwave bands, and/or in the presence of an external energy input in the form of heat.
